# EUROPEAN PATENT APPLICATION

(11) **EP 4 144 339 A2**
(43) Date of publication of application: **08.03.2023**
(21) Application number: 22192699.1
(22) Date of filing: 29.08.2022
(51) Int. Cl.: A61K 8/41, A61K 8/42, A61Q 5/12

(54) **COMBINATIONS OF AMIDO AMINE COMPOUNDS AND OLIGOESTER AMMONIUM SALTS**

(30) Priority: 03.09.2021 EP 21194925; 01.10.2021 EP 21200578
(71) Applicant: Clariant International Ltd, 4132 Muttenz (CH)
(72) Inventor: RODRIGUES, Luciana, 04795-900 São Paulo SP (BR); OKUTA, Daniela Veloso, 04795-900 São Paulo SP (BR); MASETTO, Júlia Tubone Yamashita, 04795-900 São Paulo SP (BR)
(74) Representative: Kampen, Daniela

(57) **Abstract**

The present invention relates to a combination of an amido amine compound and an oligoester ammonium salt as well as the use of said combination for hair care and/or skin care.

## Description

The present invention relates to a combination of an amido amine compound and an oligoester ammonium salt as well as the use of said combination for hair care and/or skin care.

Cosmetic products, such as hair and/or skin care products, play an important role in hygiene, beauty and well-being. For example, hair care products may be used as hair conditioner, to achieve good combability, detangling, frizz control, shape control, curl definition, to straighten hair, moisturize and nourish hair, protect hair against oxidative and other environmental stress, make hair shiny, etc. Skin care products may, for example, be used for moisturizing and nourishing skin, protecting skin against oxidative and other environmental stress, promoting skin elasticity, as anti-wrinkle agent, etc.

There is a general desire for cosmetic products, such as hair and/or skin care products. In particular, there is a desire for cosmetic products which meet the above needs of customers. For example, there is a desire for hair conditioners which effectively facilitate detangling and combing. Furthermore, there is a desire for cosmetic products which are biodegradable.

Surprisingly, it was found that combinations of an amido amine compound and an oligoester ammonium salt are particularly useful for hair and/or skin care. The combinations show beneficial effects over the individual components alone.

The present invention relates to a combination, comprising
(A) a compound of Formula (X) or a salt thereof 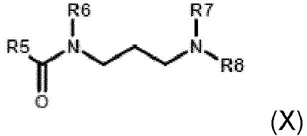 wherein
   R⁵ is selected from linear or branched C₅-C₂₃ alkyl and linear or branched C₅-C23 alkenyl;
   R⁶ is H or linear or branched C₁-C₄ alkyl;
   R⁷ is H or linear or branched C₁-C₄ alkyl;
   R⁸ is H or linear or branched C₁-C₄ alkyl; and
(B) an oligoester ammonium salt.

Advantageously, the combinations can be used for both hair care and skin care. For example, the combinations and formulations comprising them can be used to moisturize and nourish hair as well as skin. Furthermore, the combinations and corresponding formulations are useful for conditioning hair, in particular they effectively facilitate combing and detangling. The combinations and corresponding formulations are also useful for frizz control and curl definition. Advantageously, the compounds used in the present invention are biodegradable.

The combination of the present invention comprises a compound of Formula (X) or a salt thereof.

Preferably, R⁵ in Formula (X) is selected from linear or branched C₇-C₂₁ alkyl and linear or branched C₇-C₂₁ alkenyl, more preferably from linear or branched C₁₁-C₁₉ alkyl and linear or branched C₁₁-C₁₉ alkenyl, even more preferably from linear or branched C₁₅-C₁₉ alkyl and linear or branched C₁₅-C₁₉ alkenyl.

Preferably, R⁵ in Formula (X) is linear or branched C₇-C₂₁ alkyl, more preferably linear or branched C₁₁-C₁₉ alkyl, even more preferably linear or branched C₁₅-C₁₉ alkyl.

Preferably, R⁶ in Formula (X) is H.

Preferably, R⁷ in Formula (X) is methyl.

Preferably, R⁸ in Formula (X) is methyl.

In a preferred embodiment,
R⁵ in Formula (X) is selected from linear or branched C₁₇ alkyl and linear or branched C₁₇ alkenyl, preferably is linear or branched C₁₇ alkyl, particularly preferably is linear C17 alkyl;
R⁶ in Formula (X) is H;
R⁷ in Formula (X) is methyl; and
R⁸ in Formula (X) is methyl.

In a particularly preferred embodiment, the compound of Formula (X) is Stearamidopropyl Dimethylamine or N-[3-(dimethylamino)propyl]octadecanamide. Such a compound is commercially available, e.g. from Clariant as Genamin^{®} SPA.

Typically, a salt of a compound of Formula (X) is a cosmetically acceptable salt. A salt of a compound of Formula (X) may, for example, be generated in situ when the compound of Formula (X) is subjected to acidic conditions, for example an acidic environment.

Preferred embodiments of the compound of Formula (X) or a salt thereof described herein apply to the combination of the present invention as well as to all other aspects of the present invention, such as the cosmetic formulation of the present invention or the uses of the present invention.

The combination of the present invention comprises an oligoester ammonium salt.

In a preferred embodiment, the oligoester ammonium salt is obtainable by the following steps:
(i) heating a mixture of the following compounds of Formulae (I), (II), (III) and (IV) under continuous removal of reaction water:
   0.5 to 3.0 molar equivalents, preferably 0.75 to 3.0 molar equivalents, of a diethanolamine compound of Formula (I) 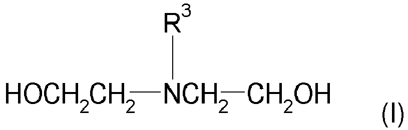 wherein R³ is linear or branched C₁-C₆-alkyl, preferably linear or branched C₁-C₄-alkyl, more preferably methyl or ethyl;
   0.5 to 1.5 molar equivalents of a dicarboxylic acid of Formula (II) 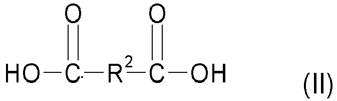
   wherein R² is linear or branched C₁-C₁₀-alkylene or linear or branched C₂-C₁₀-alkenylene, preferably linear or branched C₂-C₈-alkylene, more preferably linear or branched C₄-alkylene;
   0.5 to 1.5 molar equivalents of an organic triol (III) of Formula (111-1) or (III-2) 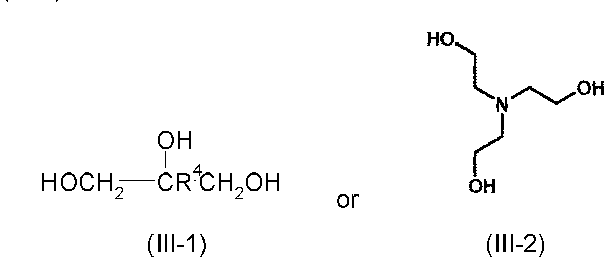 wherein R⁴ is hydrogen or linear or branched C₁-C₄-alkyl or hydroxyl-Ci-C₄-alkyl, preferably hydrogen, methyl or ethyl, more preferably hydrogen;
   1.0 molar equivalent of a monocarboxylic acid of Formula (IV)

      R¹-COOH (IV)
   wherein R¹ is linear or branched C₁₁-C₂₅-alkyl or linear or branched C₁₁-C₂₅-alkenyl, preferably linear or branched C₁₁-C₂₃-alkyl or linear or branched C₁₁-C₂₃-alkenyl, more preferably linear or branched C₁₉-C₂₃-alkyl;
(ii) reacting the oligoester product of step (i) with a quaternization agent (V), preferably dimethyl sulfate, diethyl sulfate or an alkyl halide; and
(iii) optionally purifying the oligoester ammonium salt (OAS).

In a particularly preferred embodiment,
the diethanolamine compound (I) is N-methyl diethanolamine,
the dicarboxylic acid (II) is adipic acid or sebacic acid,
the organic triol (III) is glycerol or triethanolamine, and
the monocarboxylic acid (IV) is behenic acid.

Preferred OAS are obtainable by the above steps using N-methyl diethanolamine as the diethanolamine compound (I).

Preferred OAS are obtainable by the above steps using a dicarboxylic acid (II) which is selected from the group consisting of adipic acid, sebacic acid, glutaric acid, succinic acid, itaconic acid, maleic acid, and combinations thereof. Particularly preferred OAS are obtainable by the above steps using adipic acid as the dicarboxylic acid (II). Also particularly preferred OAS are obtainable by the above steps using sebacic acid as the dicarboxylic acid (II).

Preferred OAS are obtainable by the above steps using an organic triol (III) which is selected from the group consisting of glycerol, triethanolamine, and combinations thereof. Particularly preferred OAS are obtainable by the above steps using glycerol as the organic triol (III). Also particularly preferred OAS are obtainable by the above steps using triethanolamine as the organic triol (III).

Preferred OAS are obtainable by the above steps using a monocarboxylic acid (IV) selected from the group consisting of behenic acid, oleic acid, coconut fatty acid, linoleic acid, and combinations thereof. Particularly preferred OAS are obtainable by the above steps using behenic acid as the monocarboxylic acid (IV).

In a particularly preferred embodiment, the oligoester ammonium salt (OAS) is obtainable by the above steps using N-methyl diethanolamine as the diethanolamine compound (I), adipic acid as the dicarboxylic acid (II), glycerol as the organic triol (III), and behenic acid as the monocarboxylic acid (IV).

In a particularly preferred embodiment, the oligoester ammonium salt (OAS) is obtainable by the above steps using N-methyl diethanolamine as the diethanolamine compound (I), adipic acid as the dicarboxylic acid (II), triethanolamine as the organic triol (III), and behenic acid as the monocarboxylic acid (IV).

In a particularly preferred embodiment, the oligoester ammonium salt (OAS) is obtainable by the above steps using N-methyl diethanolamine as the diethanolamine compound (I), sebacic acid as the dicarboxylic acid (II), glycerol as the organic triol (III), and behenic acid as the monocarboxylic acid (IV).

In a particularly preferred embodiment, the oligoester ammonium salt (OAS) is obtainable by the above steps using N-methyl diethanolamine as the diethanolamine compound (I), sebacic acid as the dicarboxylic acid (II), triethanolamine as the organic triol (III), and behenic acid as the monocarboxylic acid (IV).

Preferred OAS are obtainable by the above steps using a quaternization agent (V) selected from the group consisting of dimethyl sulfate, diethyl sulfate, methyl chloride, ethyl chloride, butyl chloride, and combinations thereof. Particularly preferred OAS are obtainable by the above steps using dimethyl sulfate as the quaternization agent (V).

Preferred OAS are obtainable by the above steps, wherein the molar ratio of the compounds of Formulae (I), (II), (III) and (IV) is chosen such that the molar equivalents of hydroxyl functions are in excess of the molar equivalents of acid functions.

Preferred OAS are obtainable by the above steps, wherein in step (i) the mixture of the compounds of Formulae (I), (II), (III) and (IV) is heated to a temperature from 80 to 220°C, preferably from 150 to 210°C, more preferably from 160 to 200°C.

Preferred OAS have a molecular mass Mn (number average) of from 500 to 5000 g/mol, preferably from 1000 to 4000 g/mol, for example from 1000 to 2000 g/mol, or for example from 2000 to 3000 g/mol.

Oligoester ammonium salts (OAS) and their preparation are further described in WO 2017/097816, WO 2017/097817 and WO 2017/097819. In a particularly preferred embodiment, the oligoester ammonium salt (OAS) is Polyquaternium-116. Such an OAS is commercially available, e.g. from Clariant in Genadvance^{®} Life.

Preferred embodiments of the oligoester ammonium salt described herein apply to the combination of the present invention as well as to all other aspects of the present invention, such as the cosmetic formulation of the present invention or the uses of the present invention.

In a preferred embodiment, the weight ratio of (A) the compound of Formula (X) or a salt thereof to (B) an oligoester ammonium salt is from 5:1 to 1:5, preferably from 3:1 to 1:3, more preferably from 2:1 to 1:2, particularly preferably from 1.5:1 to 1:1.5, for example 1:1. This applies to the combination of the present invention as well as to all other aspects of the present invention, such as the cosmetic formulation of the present invention or the uses of the present invention.

In a preferred embodiment, the combination of the present invention is a blend.

In a preferred embodiment, the blend comprises
from 20 to 80 wt.-%, preferably from 25 to 75 wt.-%, more preferably from 30 to 70 wt.-%, particularly preferably from 40 to 60 wt.-%, of (A) the compound of Formula (X) or a salt thereof, based on the total weight of the blend; and
from 20 to 80 wt.-%, preferably from 25 to 75 wt.-%, more preferably from 30 to 70 wt.-%, particularly preferably from 40 to 60 wt.-%, of (B) the oligoester ammonium salt, based on the total weight of the blend.

The present invention also relates to a cosmetic formulation comprising
(A) a compound of Formula (X) or a salt thereof 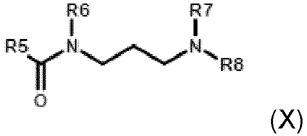 wherein
   R⁵ is selected from linear or branched C₅-C₂₃ alkyl and linear or branched C₅-C23 alkenyl;
   R⁶ is H or linear or branched C₁-C₄ alkyl;
   R⁷ is H or linear or branched C₁-C₄ alkyl;
   R⁸ is H or linear or branched C₁-C₄ alkyl; and
(B) an oligoester ammonium salt.

Preferred embodiments of the compound of Formula (X) or a salt thereof are described further above. Preferred embodiments of the oligoester ammonium salt are described further above.

In a preferred embodiment, the cosmetic formulation of the present invention comprises
from 0.1 to 25 wt.-%, preferably from 0.2 to 10 wt.-%, more preferably from 0.3 to 5 wt.-%, even more preferably from 0.4 to 3 wt.-%, particularly preferably from 0.5 to 2 wt.-%, of (A) the compound of Formula (X) or a salt thereof, based on the total weight of the cosmetic formulation; and
from 0.1 to 25 wt.-%, preferably from 0.2 to 10 wt.-%, more preferably from 0.3 to 5 wt.-%, even more preferably from 0.4 to 3 wt.-%, particularly preferably from 0.5 to 2 wt.-%, of (B) the oligoester ammonium salt, based on the total weight of the cosmetic formulation.

In a preferred embodiment, the cosmetic formulation of the present invention further comprises one or more components selected from the group consisting of emollients, waxes, emulsifiers, co-emulsifiers, solubilizers, surfactants, cationic polymers, film formers, superfatting agents, refatting agents, foam stabilizers, stabilizers, active biogenic substances, preservatives, preservation boosting ingredients, anti-fungal substances, anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, deodorants or anti-perspirants, viscosity modifiers, thickening or gelling agents, pH adjusting agents, buffering agents, anti-oxidants, chelants, astringents, sunscreens, sun protection agents, UV filters, conditioning agents, humectants, occlusive agents, pediculocides, anti-foaming agents, flavoring agents, electrolytes, oxidizing agents and reducing agents.

In a preferred embodiment, the cosmetic formulation of the present invention is selected from the group consisting of shampoo (including liquid shampoo, solid shampoo), hair conditioner (including liquid hair conditioner, solid hair conditioner), skin conditioner, body wash, facial cleanser, face mask, bubble bath, intimate wash, bath oil, cleansing milk, micellar water, make-up remover, cleansing wipes, hair mask, perfume, liquid soap, shaving soap, shaving foam, cleansing foam, day cream, anti-ageing cream, body milk, body lotion, body mousse, face serum, eye cream, sunscreen lotion, sun cream, face cream, after-shave lotion, pre-shaving cream, depilatory cream, skin-whitening gel, self-tanning cream, anti-acne gel, mascara, foundation, primer, concealer, blush, bronzer, blemish balm (bb) cream, eyeliner, night cream, eye brow gel, highlighter, lip stain, hand sanitizer, hair oil, nail varnish remover, hair styling gel, hair styling cream, anti-frizz serum, scalp treatment, hair colorant, split end fluid, deodorant, antiperspirant, baby cream, insect repellent, hand cream, sunscreen gel, foot cream, exfoliator, body scrub, cellulite treatment, bar soap, cuticle cream, lip balm, hair treatment, eye shadow, bath additive, body mist, eau de toilette, lubricating gel, moisturizer, serum, toner, aqua sorbet, cream gel, styling mousse, dry shampoo, lip stick, lip gloss, body oil, shower milk, illuminator, lip crayon, hair spray, combing cream, sunblock, ointment, and lipstick.

In a particularly preferred embodiment, the cosmetic formulation of the present invention is a hair conditioner or shampoo. The hair conditioner may, for example, be a liquid hair conditioner or a solid hair conditioner. The shampoo may, for example, be a liquid shampoo or a solid shampoo. The hair conditioner or the shampoo may, for example, be a rinse-off product or a leave-on product.

The present invention also relates to the use of the combination of the present invention for hair care and/or skin care. Preferably, the combination of the present invention is used for hair care. Particularly preferably, the combination of the present invention is used as a hair conditioning agent.

The present invention also relates to the use of the cosmetic formulation of the present invention as a hair care and/or skin care formulation. Preferably, the cosmetic formulation of the present invention is used as a hair care formulation. Particularly preferably, the cosmetic formulation of the present invention is used as a hair conditioner or shampoo. The hair conditioner may, for example, be a liquid hair conditioner or a solid hair conditioner. The shampoo may, for example, be a liquid shampoo or a solid shampoo. The hair conditioner or the shampoo may, for example, be a rinse-off product or a leave-on product.

In a preferred embodiment, the combination of the present invention or the cosmetic formulation of the present invention may be used as hair conditioner, to achieve good combability, detangling, frizz control, shape control, curl definition, to straighten hair, moisturize and nourish hair, protect hair against oxidative and other environmental stress, or make hair shiny.

In a preferred embodiment, the combination of the present invention or the cosmetic formulation of the present invention may be used for moisturizing and nourishing skin, protecting skin against oxidative and other environmental stress, promoting skin elasticity, or as anti-wrinkle agent.

### Examples

### Examples 1 to 7 - Combability

The following formulations were prepared (amounts are indicated in wt.-%) and the dry combing force was measured:

| **Example** | **1 (CE)** | **2 (CE)** | **3 (CE)** | **4 (CE)** | **5 (INV)** | **6 (CE)** | **7 (INV)** |
|---|---|---|---|---|---|---|---|
| CTAC | 2 | - | - | - | - | - | - |
| BTAC | - | 2 | - | 1 | - | 1.5 | - |
| Genamin^{®} SPA | - | - | 2 | - | 1 | - | 1.5 |
| Genadvance^{®} Life | - | - | - | 1 | 1 | 0.5 | 0.5 |
| C16-18 fatty alcohol | 4 | 4 | 4 | 4 | 4 | 4 | 4 |
| Preservative system | 0.1 | 0.1 | 0.1 | 0.1 | 0.1 | 0.1 | 0.1 |
| Disodium EDTA | 0.1 | 0.1 | 0.1 | 0.1 | 0.1 | 0.1 | 0.1 |
| pH adjusted to | 4 to 5 | 4 to 5 | 4 to 5 | 4 to 5 | 4 to 5 | 4 to 5 | 4 to 5 |
| Water to | 100 | 100 | 100 | 100 | 100 | 100 | 100 |
| Dry combing force [millijoules, mJ] | 8.0 | 7.7 | 7.1 | 7.1 | 5.9 | 7.0 | 6.7 |

### Key:

CE = comparative example
INV = example according to the invention
CTAC: cetyltrimethylammonium chloride
BTAC: benzyltrimethylammonium chloride
Genamin^{®} SPA (Clariant): Stearamidopropyl Dimethylamine
Genadvance^{®} Life (Clariant): Polyquaternium-116 (and) Butylene Glycol

### Dry combing force measurement:

The dry combing force was measured using a Dia-Stron MTT175 instrument. Caucasian hair tresses (bleached) were washed, and excess water was removed by running the hair tresses gently between two fingers from top to bottom. The formulations according to Examples 1 to 7 were applied to the hair tresses (distributed homogeneously and left on for 30 seconds). The hair tresses were rinsed with water. Excess water was removed by running the hair tresses gently between two fingers from top to bottom. The formulations according to Examples 1 to 7 were applied to the hair tresses two more times. The hair tresses were left to dry in a controlled environment (temperature: 25 ± 2 °C; relative humidity: 40 ± 5%). The hair tresses were pre-combed three times before the combing force measurement. The hair tresses were added to the equipment and the dry combing force was measured. The dry combing force is the energy required to dry comb the hair tresses.

### Conclusions:

The combinations and formulations according to the invention (Examples 5 and 7) are beneficial over the amido amine compound alone (Comparative Example 3) or benchmarks (Examples 1 and 3). Furthermore, a combination of the oligoester ammonium salt with an amido amine compound (Examples 5 and 7) is beneficial over a combination of the oligoester ammonium salt with BTAC (Comparative Examples 4 and 6).

The combinations and formulations of the present invention are useful for detangling and conditioning hair as well as for improving and facilitating combing.

## Claims

1. A combination, comprising
(A) a compound of Formula (X) or a salt thereof 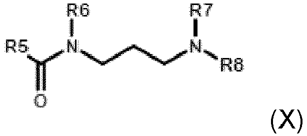 wherein
R⁵ is selected from linear or branched C₅-C₂₃ alkyl and linear or branched C₅-C₂₃ alkenyl;
R⁶ is H or linear or branched C₁-C₄ alkyl;
R⁷ is H or linear or branched C₁-C₄ alkyl;
R⁸ is H or linear or branched C₁-C₄ alkyl; and
(B) an oligoester ammonium salt.

2. The combination according to claim 1, wherein the oligoester ammonium salt is obtainable by the following steps:
(i) heating a mixture of the following compounds of Formulae (I), (II), (III) and (IV) under continuous removal of reaction water:
0.5 to 3.0 molar equivalents, preferably 0.75 to 3.0 molar equivalents, of a diethanolamine compound of Formula (I) 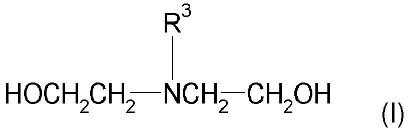 wherein R³ is linear or branched C₁-C₆-alkyl, preferably linear or branched C₁-C₄-alkyl, more preferably methyl or ethyl;
0.5 to 1.5 molar equivalents of a dicarboxylic acid of Formula (II) 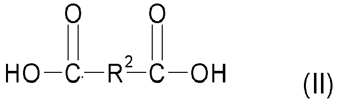 wherein R² is linear or branched C₁-C₁₀-alkylene or linear or branched C₂-C₁₀-alkenylene, preferably linear or branched C₂-C₈-alkylene, more preferably linear or branched C₄-alkylene;
0.5 to 1.5 molar equivalents of an organic triol (III) of Formula (111-1) or (III-2) 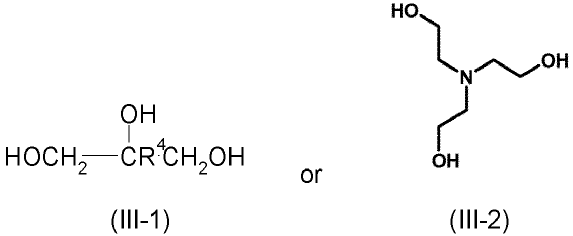 wherein R⁴ is hydrogen or linear or branched C₁-C₄-alkyl or hydroxyl-Ci-C₄-alkyl, preferably hydrogen, methyl or ethyl, more preferably hydrogen;
1.0 molar equivalent of a monocarboxylic acid of Formula (IV)
R¹-COOH (IV)
wherein R¹ is linear or branched C₁₁-C₂₅-alkyl or linear or branched C₁₁-C₂₅-alkenyl, preferably linear or branched C₁₁-C₂₃-alkyl or linear or branched C₁₁-C₂₃-alkenyl, more preferably linear or branched C₁₉-C₂₃-alkyl;
(ii) reacting the oligoester product of step (i) with a quaternization agent (V), preferably dimethyl sulfate, diethyl sulfate or an alkyl halide; and
(iii) optionally purifying the oligoester ammonium salt (OAS).

3. The combination according to claim 1 or 2, wherein
R⁵ in Formula (X) is selected from linear or branched C₁₇ alkyl and linear or branched C₁₇ alkenyl, preferably is linear or branched C₁₇ alkyl, particularly preferably is linear C₁₇ alkyl;
R⁶ in Formula (X) is H;
R⁷ in Formula (X) is methyl; and
R⁸ in Formula (X) is methyl.

4. The combination according to any of claims 1 to 3, wherein
the diethanolamine compound (I) is N-methyl diethanolamine,
the dicarboxylic acid (II) is adipic acid or sebacic acid,
the organic triol (III) is glycerol or triethanolamine, and
the monocarboxylic acid (IV) is behenic acid.

5. The combination according to any of claims 1 to 4, wherein the weight ratio of (A) the compound of Formula (X) or a salt thereof to (B) an oligoester ammonium salt is from 5:1 to 1:5, preferably from 3:1 to 1:3, more preferably from 2:1 to 1:2, particularly preferably from 1.5:1 to 1:1.5, for example 1:1.

6. The combination according to any of claims 1 to 5, wherein the combination is a blend.

7. The combination according to claim 6, wherein the blend comprises from 20 to 80 wt.-%, preferably from 25 to 75 wt.-%, more preferably from 30 to 70 wt.-%, particularly preferably from 40 to 60 wt.-%, of (A) the compound of Formula (X) or a salt thereof, based on the total weight of the blend; and from 20 to 80 wt.-%, preferably from 25 to 75 wt.-%, more preferably from 30 to 70 wt.-%, particularly preferably from 40 to 60 wt.-%, of (B) the oligoester ammonium salt, based on the total weight of the blend.

8. A cosmetic formulation comprising
(A) a compound of Formula (X) or a salt thereof 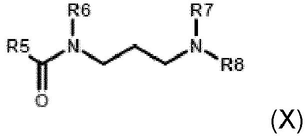 wherein
R⁵ is selected from linear or branched C₅-C₂₃ alkyl and linear or branched C₅-C₂₃ alkenyl;
R⁶ is H or linear or branched C₁-C₄ alkyl;
R⁷ is H or linear or branched C₁-C₄ alkyl;
R⁸ is H or linear or branched C₁-C₄ alkyl; and
(B) an oligoester ammonium salt.

9. The cosmetic formulation according to claim 8, wherein the compound of Formula (X) or a salt thereof is defined as in claim 3 and/or wherein the oligoester ammonium salt is defined in claim 2 or 4

10. The cosmetic formulation according to claim 8 or 9, wherein the cosmetic formulation comprises
from 0.1 to 25 wt.-%, preferably from 0.2 to 10 wt.-%, more preferably from 0.3 to 5 wt.-%, even more preferably from 0.4 to 3 wt.-%, particularly preferably from 0.5 to 2 wt.-%, of (A) the compound of Formula (X) or a salt thereof, based on the total weight of the cosmetic formulation; and
from 0.1 to 25 wt.-%, preferably from 0.2 to 10 wt.-%, more preferably from 0.3 to 5 wt.-%, even more preferably from 0.4 to 3 wt.-%, particularly preferably from 0.5 to 2 wt.-%, of (B) the oligoester ammonium salt, based on the total weight of the cosmetic formulation.

11. The cosmetic formulation according to any of claims 8 to 10, wherein the cosmetic formulation further comprises one or more components selected from the group consisting of emollients, waxes, emulsifiers, co-emulsifiers, solubilizers, surfactants, cationic polymers, film formers, superfatting agents, refatting agents, foam stabilizers, stabilizers, active biogenic substances, preservatives, preservation boosting ingredients, anti-fungal substances, anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, deodorants or anti-perspirants, viscosity modifiers, thickening or gelling agents, pH adjusting agents, buffering agents, anti-oxidants, chelants, astringents, sunscreens, sun protection agents, UV filters, conditioning agents, humectants, occlusive agents, pediculocides, anti-foaming agents, flavoring agents, electrolytes, oxidizing agents and reducing agents.

12. The cosmetic formulation according to any of claims 8 to 11, wherein the cosmetic formulation is selected from the group consisting of shampoo (including liquid shampoo, solid shampoo), hair conditioner (including liquid hair conditioner, solid hair conditioner), skin conditioner, body wash, facial cleanser, face mask, bubble bath, intimate wash, bath oil, cleansing milk, micellar water, make-up remover, cleansing wipes, hair mask, perfume, liquid soap, shaving soap, shaving foam, cleansing foam, day cream, anti-ageing cream, body milk, body lotion, body mousse, face serum, eye cream, sunscreen lotion, sun cream, face cream, after-shave lotion, pre-shaving cream, depilatory cream, skin-whitening gel, self-tanning cream, anti-acne gel, mascara, foundation, primer, concealer, blush, bronzer, blemish balm (bb) cream, eyeliner, night cream, eye brow gel, highlighter, lip stain, hand sanitizer, hair oil, nail varnish remover, hair styling gel, hair styling cream, anti-frizz serum, scalp treatment, hair colorant, split end fluid, deodorant, antiperspirant, baby cream, insect repellent, hand cream, sunscreen gel, foot cream, exfoliator, body scrub, cellulite treatment, bar soap, cuticle cream, lip balm, hair treatment, eye shadow, bath additive, body mist, eau de toilette, lubricating gel, moisturizer, serum, toner, aqua sorbet, cream gel, styling mousse, dry shampoo, lip stick, lip gloss, body oil, shower milk, illuminator, lip crayon, hair spray, combing cream, sunblock, ointment, and lipstick.

13. Use of a combination as defined in any of claims 1 to 7 for hair care and/or skin care, preferably for hair care, particularly preferably as a hair conditioning agent.

14. Use of a cosmetic formulation as defined in any of claims 8 to 12 as a hair care and/or skin care formulation, preferably as a hair care formulation, particularly preferably as a hair conditioner (including liquid hair conditioner, solid hair conditioner) or shampoo (including liquid shampoo, solid shampoo).
